# EUROPÄISCHE PATENTSCHRIFT

(11) **EP 3 740 114 B1**
(45) Veröffentlichungstag und Bekanntmachung des Hinweises auf die Patenterteilung: **23.03.2022**
(21) Anmeldenummer: 19700927.7
(22) Anmeldetag: 17.01.2019
(51) Int. Cl.: A61B 3/10

(54) **VERFAHREN ZUR DURCHGEHENDEN KONTROLLE DER FIXATION EINES PATIENTENAUGES WÄHREND DER ERFASSUNG DESSEN BIOMETRISCHER MESSDATEN**
METHOD FOR THE CONTINUOUS CONTROL OF THE FIXATION OF A PATIENT'S EYE DURING THE DETECTION OF BIOMETRIC MEASUREMENT DATA OF THE PATIENT'S EYE
PROCÉDÉ POUR LE CONTRÔLE CONTINU DE LA FIXATION D'UN OEIL D'UN PATIENT PENDANT LA DÉTECTION DE SES DONNÉES DE MESURE BIOMÉTRIQUES

(30) Priorität: 19.01.2018 DE 102018200829
(43) Veröffentlichungstag der Anmeldung: 25.11.2020
(73) Patentinhaber: Carl Zeiss Meditec AG, 07745 Jena (DE)
(72) Erfinder: BAJRAMOVIC, Ferid, 94437 Mamming (DE); CHEN, Wei-Jun, 07751 Jena (DE); BÜHREN, Tobias, 89073 Ulm (DE)
(74) Vertreter: Kintzel, Klaus-Peter
(86) Internationale Anmeldenummer: PCT/EP2019/051090
(87) Internationale Veröffentlichungsnummer: WO 2019/141750

(56) Entgegenhaltungen:
- WO-A1-2015/116981
- US-B2- 9 492 079

## Beschreibung

Die vorliegende Erfindung betrifft ein Verfahren zur Erfassung biometrischer Messdaten eines Patientenauges, bei dem eine Kontrolle der Fixation möglichst während der gesamten biometrischen Messung erfolgt.

Fixation ist wichtig für korrekte Datenaufnahme bzw. Messungen bei ophthalmologischen Diagnose- und Therapiegeräten, insbesondere in der optischen Biometrie, Keratometrie und Topographie. Allerdings ist eine durchgehend perfekte Fixation selbst bei kooperativen, gesunden Patienten unrealistisch, da Augenbewegungen trotz unterschiedlicher Fixiermarken nicht zu vermeiden sind.

Inwieweit sich diese aus den Augenbewegungen resultierenden Fixationsfehler auf die Messergebnisse auswirken, hängt vom jeweiligen Messverfahren ab, da diese unterschiedliche Toleranzen für Augenbewegung aufweisen.

Bei vielen Patienten wird Augenbewegung dazu führen, dass Fixationsfehler die Toleranz des jeweiligen Messverfahrens überschreitet. Insbesondere bei Patienten mit eingeschränktem Sehvermögen kann die Fixation durchgehend zu schlecht sein. Bei Messungen der optischen Biometrie beispielsweise können Messfehler durch fehlende Fixation zu einer suboptimal ermittelten Brechkraft der zu implantierenden IOL und letztlich zu einer Verschlechterung der Refraktion des Patienten nach Operation des grauen Stars führen.

Nach dem bekannten Stand der Technik sind zum einen Lösungen bekannt um die Aufmerksamkeit eines Patienten möglichst hoch zu halten, um ungewollte Augenbewegungen zu vermeiden oder zumindest zu minimieren.

In der DE 10 2009 007 732 A1 wird beispielsweise eine Anordnung zur Darstellung einer Fixiermarke für ophthalmologische Untersuchungs- und/oder Behandlungsgeräte beschrieben. Hierbei wird die dem Patienten angebotene Fixiermarke so modifiziert, dass die entstehende Strahlstruktur energetisch und/oder zeitlich und/oder örtlich und/oder spektral veränderbar ist. Mit der beweglichen Fixiermarke wird erreicht, dass der Patient sein Auge auf diese ausrichtet und diesem problemlos, d. h. ohne größere Ansprüche an seine Konzentrationsfähigkeit folgen kann.

Es hat sich gezeigt, dass derartige Lösungen lediglich für zeitlich kurze Untersuchungen bzw. Messungen am Auge geeignet sind. Dauern diese jedoch länger, kann es selbst bei kooperativen, gesunden Patienten zu ungewollten Augenbewegungen kommen.

Zum anderen sind nach dem bekannten Stand der Technik Lösungen bekannt, bei denen eine absolute Fixationsprüfung und/oder ein kontrolliertes Umfixieren des Patienten erfolgt.

Hierzu beschreibt die DE 10 2012 019 473 A1 beispielsweise ein Verfahren zur verlässlichen Bestimmung der Achslänge eines Auges mittels optischer Kohärenztomographie (OCT), wobei hierbei die Ausrichtung des Messgeräts zum Auge für alle ein- oder zweidimensionalen Scans kontrolliert wird um eine verlässliche Bestimmung der Achslänge des Auges gewährleisten zu können. Insbesondere werden nach dem Ausrichten der Sehachse des Auges auf die Haupt-Messachse des Messgerätes B-Scans realisiert, aus denen retinale Gewebestrukturen detektiert werden um die Achslängen zu bestimmen, die dann zur Detektion der Fovea verwendet werden, um die Ausrichtung zu kontrollieren. Die aus den B-Scans ermittelten Achslängen werden dann in Abhängigkeit von der ermittelten Position der Fovea bzw. deren lateralen Abstand zur optischen Achse des Messgerätes bestätigt bzw. korrigiert und ausgegeben.

Nachteilig wirkt sich bei der hier vorgeschlagenen Lösung aus, dass sich die Fixationsinformationen nicht auf andere Zeitpunkte übertragen lassen.

In der US 9 492 079 B2 wird ein Verfahren zur durchgehenden Kontrolle der Fixation eines Patientenauges während der Erfassung dessen biometrischer Messdaten beschrieben, wobei ein zentraler retinaler OCT-Scan mit absoluter Fixationsinformation aufgenommen wird und anhand dessen der Fixationszustand geprüft wird.

Der vorliegenden Erfindung liegt die Aufgabe zugrunde eine Lösung zu entwickeln, bei der eine Fixationskontrolle als Teil der Messdatenaufnahme oder parallel dazu möglich ist, um die Qualität aufgenommener Messdaten zu prüfen und idealerweise auch um Fixationsfehler zu kompensieren. Dabei sollte die Fixationsprüfung und/oder Fixationskompensation möglichst durchgehend erfolgen und zwar ohne, dass dadurch die Messdatenaufnahme wesentlich verlängert wird.

Diese Aufgabe wird mit dem Verfahren zur durchgehenden Kontrolle der Fixation eines Patientenauges während der Erfassung dessen biometrischer Messdaten, erfindungsgemäß durch folgende Verfahrensschritte gelöst:
a) aufnehmen eines zentralen retinalen OCT-Scans mit absoluter Fixationsinformation,
b) aufnehmen eines Frontalbildes mit relativer Fixationsinformation gleichzeitig bzw. unmittelbar vor oder nach dem zentralen retinalen OCT-Scan, bei zumindest teilweise diffuser Beleuchtung,
c) zeitlich möglichst durchgehend aufnehmen weiterer Frontalbilder mit relativer Fixationsinformation,
d) prüfen des Fixationszustandes anhand des zentralen retinalen OCT-Scans,
e) bei Vorliegen einer korrekten Fixation wird diese auch für das im Verfahrensschritt b) aufgenommene Frontalbild angenommen,
f) berechnen eines, die Abweichungen zu dem im Verfahrensschritt b) aufgenommen Frontalbild beschreibenden, Differenzvektors für jedes im Verfahrensschritt c) aufgenommene Frontalbild,
g) vergleichen der berechneten Differenzvektoren der im Verfahrensschritt c) aufgenommenen Frontalbilder mit einem definierten Schwellwert, wobei
h) bei unterschrittenen Schwellwerten für die aufgenommenen Frontalbilder eine korrekte Fixation und bei überschrittenen keine korrekte Fixation angenommen wird und
i) nur die biometrischen Messdaten verwendet werden, die kurz vor, während oder kurz nach den Frontalbildern mit korrekter Fixation erfasst wurden.

Bevorzugte Weiterbildungen und Ausgestaltungen sind Gegenstand der abhängigen Ansprüche.

Das erfindungsgemäße Verfahren ist zwar insbesondere zur Erfassung biometrischer Messdaten eines Patientenauges vorgesehen, kann aber auch für andere Messaufgaben, bei denen unterschiedliche Messmodi zur Anwendung kommen und die Ausrichtung des Messobjektes für die Messergebnisse von Bedeutung sind, angewendet werden.

Die Erfindung wird nachfolgend anhand von Ausführungsbeispielen näher beschrieben. Dazu zeigen
- Figur 1:: zwei Beispiele zentraler retinaler OCT-Scans bei unterschiedlicher Fixation,
- Figur 2:: zwei Frontalbilder mit von der Hornhaut reflektierten Lichtspotmustern, bei zumindest teilweise diffuser Beleuchtung bei unterschiedlicher Fixation,
- Figur 3:: zwei Frontalbilder mit von der Hornhaut reflektierten Lichtspotmustern bei unterschiedlicher Fixation und
- Figur 4:: eine mögliche Aufnahmesequenz mit einem zentralen retinalen OCT-Scan und unterschiedlichen Frontalbildern zur durchgehenden Prüfung des Fixationszustandes.

Bei dem vorgeschlagenen Verfahren zur durchgehenden Kontrolle der Fixation eines Patientenauges während der Erfassung dessen biometrischer Messdaten, werden in Abhängigkeit der unterschiedlichen Aufnahmemodi aus bereits vorhanden bzw. zusätzlich erfasster Aufnahmen und/oder Daten Informationen zur Fixation extrahiert.

Erfindungsgemäß lässt sich das Verfahren in folgende Verfahrensschritte gliedern:
a) aufnehmen eines zentralen retinalen OCT-Scans mit absoluter Fixationsinformation,
b) aufnehmen eines Frontalbildes mit relativer Fixationsinformation gleichzeitig bzw. unmittelbar vor oder nach dem zentralen retinalen OCT-Scan, bei zumindest teilweiser, diffuser Beleuchtung,
c) zeitlich möglichst durchgehend aufnehmen weiterer Frontalbilder mit relativer Fixationsinformation,
d) prüfen des Fixationszustandes anhand des zentralen retinalen OCT-Scans,
e) bei Vorliegen einer korrekten Fixation wird diese auch für das im Verfahrensschritt b) aufgenommene Frontalbild angenommen,
f) berechnen eines, die Abweichungen zu dem im Verfahrensschritt b) aufgenommen Frontalbild beschreibenden, Differenzvektors für jedes im Verfahrensschritt c) aufgenommene Frontalbild,
g) vergleichen der berechneten Differenzvektoren der im Verfahrensschritt c) aufgenommenen Frontalbilder mit einem definierten Schwellwert, wobei
h) bei unterschrittenen Schwellwerten für die aufgenommenen Frontalbilder eine korrekte Fixation und bei überschrittenen keine korrekte Fixation angenommen wird und
i) nur die biometrischen Messdaten verwendet werden, die kurz vor, während oder kurz nach den Frontalbildern mit korrekter Fixation erfass wurden.

Im Verfahrensschritt a) wird ein zentraler retinaler OCT-Scans entlang der optischen Achse des Messgeräts realisiert. Dieser OCT-Scan mit lateralem Scan im Bereich der Retina beinhaltet absolute Fixationsinformationen und ermöglicht somit eine Kontrolle der Fixation des zu vermessenden Auges.

Wichtig ist hierbei, dass der, absolute Fixationsinformationen enthaltende, zentrale retinale OCT-Scan zu einem günstigen Zeitpunkt aufgenommen wird, zu dem mit hoher Wahrscheinlichkeit eine korrekte Fixation vorliegt.

In Abhängigkeit von der Gesamtdauer der eigentlichen biometrischen Messung ist es sinnvoll, zentrale retinale OCT-scans mehrfach, d. h. so oft wie möglich zu realisieren.

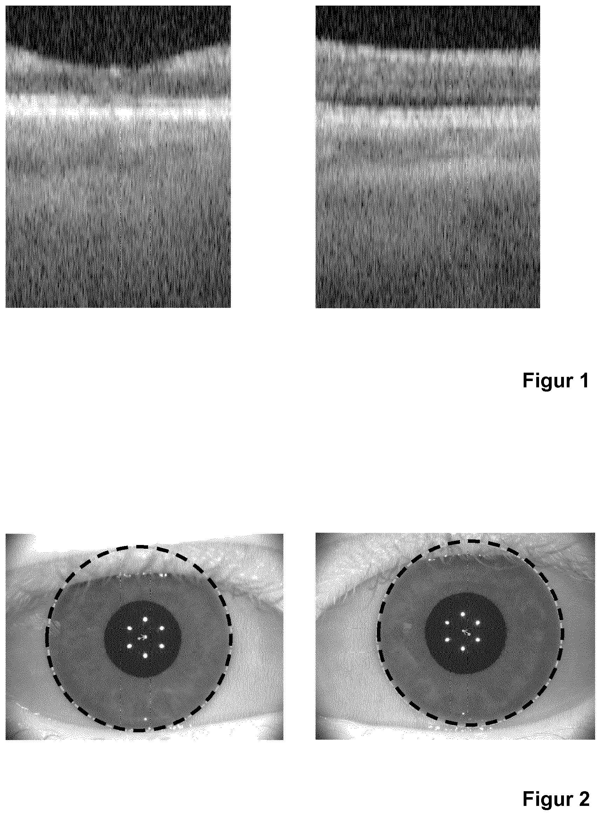

Hierzu zeigt die **Figur 1** je ein Beispiel eines solchen Scans bei korrekter und bei inkorrekter Fixation.

Da die linke Abbildung einen retinalen OCT-Scan zeigt, der die foveale Grube zentral beinhaltet, erfolgte die Aufzeichnung dieses Scans bei korrekter Fixation. Im Gegensatz dazu zeigt die rechte Abbildung einen retinalen OCT-Scan, der die foveale Grube nicht beinhaltet. Bei der Aufzeichnung dieses Scans liegt keine korrekte Fixation vor. Es könnte aber auch sein, dass die foveale Grube in der rechten Abbildung nicht zu sehen ist, da das Auge morphologische Veränderung aufweist.

Für den Fall, dass im Ergebnis des Verfahrensschrittes d) der zentrale retinale OCT-Scan die foveale Grube nicht enthält und somit keine korrekte Fixation vorliegt, erhält der Bediener einen entsprechenden Hinweis. Danach wird das Verfahren durch den Bediener oder automatisch erneut mit Verfahrensschritt a) gestartet.

Für die Aufzeichnung derartiger retinaler OCT-Scans, die auch als Fixations-Check-Scans bezeichnet werden, kann beispielsweise IOLMaster 700 der Carl Zeiss Meditec AG Verwendung finden.

Obwohl den Patienten während der Messdatenaufnahme ein Fixationsanreiz beispielsweise in Form eines Fixationslichtpunktes angeboten wird, kommt es auch bei kooperativen gesunden Patienten zu ungewollten Augenbewegungen. Deshalb ist eine Fixationskontrolle nicht nur zu einem festen Zeitpunkt, sondern durchgehend während der gesamten Messdatenaufnahme wichtig.

Augenbewegung bestehen im Allgemeinen aus Rotation und Translation im dreidimensionalen Raum. Je nach verfügbaren Datenaufnahmemodi können unterschiedliche dieser sechs Freiheitsgrade ermittelt werden.

Es kann aber aus technischen Gründen ausgeschlossen sein, parallel zur Messdatenaufnahme stets auch zentrale retinale OCT-Scans aufzunehmen. Eine zeitlich engmaschige abwechselnde Aufnahme kann technisch durch die Umschaltzeit zwischen verschiedenen Datenaufnahmemodi begrenzt sein.

Auch kann die Aufnahmedauer dieser Scans die Gesamtdauer der Messdatenaufnahme ungünstig verlängern.

Erfindungsgemäß wird im Verfahrensschritt b) ein Frontalbild mit relativer Fixationsinformation gleichzeitig bzw. unmittelbar vor oder nach dem zentralen retinalen OCT-Scan, bei zumindest teilweise diffuser Beleuchtung aufgenommen.

Aus diesem Frontalbild lässt sich beispielsweise die laterale Translation, als Verschiebung der Strukturen im Bild ermitteln. In Abhängigkeit von der verwendeten Abbildungsoptik kann auch die axiale Translation, als Skalierung der Strukturen im Bild oder bei telezentrischer Abbildung lediglich als Defokussierung erkennbar sein.

Hierbei ist es besonders vorteilhaft, wenn für das im Verfahrensschritt b) aufzunehmende Frontalbild ein zusätzlicher Reflex auf der Kornea erzeugt wird. Dieser zusätzlich erzeugte Reflex wird bevorzugt nahe am Vertex und besonders bevorzugt am Vertex, durch Projektion eines kollimierten Lichtstrahles entlang der optischen Achse des Gerätes erzeugt.

Dieser zusätzlich erzeugte Reflex auf der Kornea erlaubt es außerdem Rotationsanteile für das sogenannte Nicken und Gieren zu ermitteln.

Für die Auswertung der relativen Fixationsinformation ist es vorteilhaft an den Limbus oder die Pupille einen Kreisring zu fitten. Dadurch lässt sich die Veränderung der Position des Lichtspots relativ zu anderen Strukturen im Bild, beispielsweise dem Mittelpunkt dieses Kreisrings zu detektieren.

Hierzu zeigt die **Figur 2** zwei Frontalbilder bei zumindest teilweiser, diffuser Beleuchtung mit an den Limbus angefittetem Kreisring und unterschiedlicher Fixation.

Insbesondere ist hierbei der Abstand zwischen Kreismittelpunkt des Limbus zum zusätzlichen Reflex auf der Kornea abhängig von der Fixationsrichtung, wie am Unterschied zwischen linker und rechter Abbildung zu erkennen ist.

Daher erlaubt es diese Modalität, zeitliche Veränderungen der Fixationsrichtung zu ermitteln. Dies resultiert aus dem Fakt, dass sich Strukturen am Auge in unterschiedlicher Tiefe bei Änderung der Fixationsrichtung im Kamerabild relativ zueinander verschieben.

Im weiteren Verfahrensverlauf werden gemäß Verfahrensschritt c) zeitlich möglichst durchgehend weitere Frontalbilder mit relativer Fixationsinformation aufgenommen. Erfindungsgemäß werden diese Frontalbilder in Abhängig der Aufnahmemodi bei unterschiedlichen Beleuchtungssituationen aufgenommen.

Hierfür ist eine schnelle Umschaltung zwischen diesen Aufnahmemodi wichtig. Dies kann beispielsweise dadurch erreicht werden, dass Kamera-basierte Aufnahmemodi allein durch Ändern der Beleuchtung umgeschaltet werden, wobei die Beleuchtungsänderung vorzugsweise mit dem Kameratrigger synchronisiert erfolgt.

Abhängig davon, welche Aufnahmemodi zeitlich parallel möglich sind und welche sich schnell oder nur langsam umschalten lassen, sind verschiedene zeitliche Anordnungen der unterschiedlichen Datenaufnahmen sinnvoll.

Beispielsweise werden hier Frontalbilder verwendet, die ein von der Hornhaut reflektiertes Lichtspot-Muster zeigen. Auch hier lassen sich bei telezentrischer Beobachtung laterale Translationsbewegungen ermitteln. Rotation aus dieser Art von Frontalbildern zu ermitteln ist im Allgemeinen unmöglich, insbesondere da die Hornhaut des Auges nahezu kugelförmig ist.

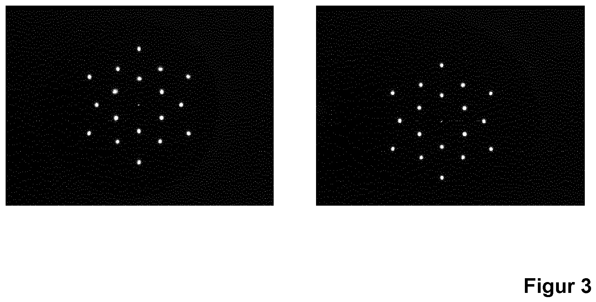

Hierzu zeigt die **Figur 3** zwei Frontalbilder mit von der Hornhaut reflektierten Lichtspotmustern. Hierbei wird das Lichtspotmuster entlang der optischen Achse des Messgerätes auf das Auge projiziert. Dem entsprechend sind den beiden Abbildungen zu entnehmen, dass nur in der linken Abbildung eine korrekte Fixation vorliegt. Das Lichtspotmuster in der rechten Abbildung ist deutlich nach unten verschoben.

Für die Aufzeichnung derartiger Frontalbilder kann beispielsweise ebenfalls ein IOLMaster 700 der Carl Zeiss Meditec AG Verwendung finden. Allerdings sind diese Frontalbilder Ergebnis der im IOLMaster 700 enthaltenen Keratometer-Anwendung.

Erfindungsgemäß erfolgt im Verfahrensschritt f) die Berechnung eines, die Abweichungen zu dem im Verfahrensschritt b) aufgenommen Frontalbild beschreibenden, Differenzvektors für jedes im Verfahrensschritt c) aufgenommene Frontalbild.

Für die Berechnung der Differenzvektoren wird der an den Limbus oder die Pupille gefittete Kreisring genutzt, wobei vorzugsweise der Abstand zwischen dem Mittelpunkt des Kreisringes und dem zusätzlich erzeugten Reflex genutzt wird.

Bei der Berechnung der Differenzvektoren ist zu berücksichtigen, dass die zu berechnenden Differenzvektoren von der Vorderkammertiefe des betreffenden Auges abhängig sind.

Je länger die Vorderkammertiefe ist, desto größer ist auch der Einfluss der Fixation auf den Offsetvektor. Dabei lässt sich der Zusammenhang einfach multiplikativ modellieren, d. h. eine doppelte Vorderkammertiefe führt zu einem verdoppelten Schwellwert.

Zwar gelten die geometrischen Verhältnisse nur näherungsweise für ein reales Auge. Der multiplikative Einfluss der Vorderkammertiefe auf die Länge des Offsetvektors stellt aber zumindest eine gute Approximation dar und erlaubt eine einfache Anpassung des Schwellwerts an das jeweilige Patientenauge anhand dessen Vorderkammertiefe.

Mit der vorgeschlagenen, erfinderischen Lösung ist eine durchgehende Prüfung des Fixationszustandes möglich, indem:
- Eine Prüfung auf korrekte Fixation anhand des zentralen retinalen OCT-Scans erfolgt, wobei dies manuell oder automatisch und passiv oder aktiv unter Mitwirkung des Patienten erfolgen kann.
- Die relativen Fixationsinformationen aus geeigneten, zu verschiedenen Zeitpunkten aufgenommenen Frontalbildern werden mit der relativen Fixationsinformation zum Aufnahmezeitpunkt des zentralen retinalen OCT-Scans verglichen, welcher absolute Fixationsinformation liefert. Dies kann beispielsweise mittels eines geeigneten Schwellwerts auf die Länge der errechneten Differenzvektoren zu den beiden zwei Aufnahmezeitpunkten erfolgen.
- Die Fixationsprüfung des zentralen retinalen OCT-Scans überträgt sich nur auf Aufnahmezeitpunkte, zu denen dieser Schwellwert nicht überschritten wird. Ist die Fixation laut zentralem retinalen OCT-Scans korrekt, so ist sie auch zu diesen Aufnahmezeitpunkten korrekt.
- In der sehr kurzen Zeit zwischen zwei Frontalbildern mit relativen Fixationsinformationen kann von identischer Fixation ausgegangen werden, falls die Länge des Differenzvektors einen geeigneten Schwellwert nicht überschreitet. Ist die Fixation laut übertragener Fixationsprüfung für die Aufnahmezeitpunkte der beiden Frontalbilder korrekt, so kann auch davon ausgegangen werden, dass sie zeitlich dazwischen korrekt ist.

Ein geeigneter Schwellwert für diese Fixationsprüfung hängt im Allgemeinen von der Messaufgabe ab, insbesondere von deren Toleranz für Fixationsabweichungen. Er kann aus einem theoretischen Augenmodell abgeleitet oder experimentell bestimmt werden.

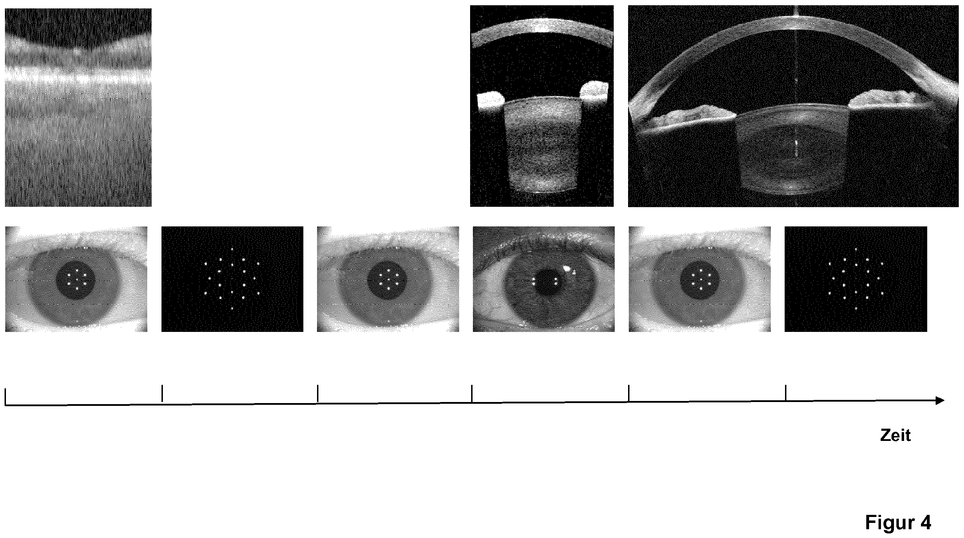

Hierzu zeigt die **Figur 4** eine mögliche Aufnahmesequenz mit einem zentralen retinalen OCT-Scan und unterschiedlichen Frontalbildern zur durchgehenden Prüfung des Fixationszustandes.

Zeitgleich zum zentralen retinalen OCT-Scan (Abbildung links oben) wurde ein erstes Frontalbild bei zumindest teilweise diffuser Beleuchtung (Abbildung links unten) aufgenommen. Danach wurden durchgehend weitere Frontalbilder bei unterschiedlichen Beleuchtungssituationen aufgenommen (Abbildungen der unteren Reihe).

Während die Abbildungen 1, 3 und 5 der unteren Reihe Frontalbilder bei zumindest teilweise diffuser Beleuchtung und einem zusätzlichen Reflex auf der Kornea zeigen, sind die Abbildungen 2 und 6 Aufnahmen der reinen Keratometrie-Anwendung. Die Abbildung 4 zeigt ein Frontalbild bei einer anderen Beleuchtungssituation, bei zumindest teilweiser, diffuser Beleuchtung, allerdings ohne zusätzlichen Reflex auf der Kornea.

In der oberen Reihe sind OCT-Aufnahmen anderer Scanmodi abgebildet. Der abgebildete Zeitstrahl soll dokumentieren, dass dargestellte Abbildungen gleichzeitig (untereinander) und nacheinander (nebeneinander) aufgenommen worden sind.

Erfindungsgemäß können bei dem vorgeschlagenen Verfahren die berechneten Differenzvektoren zur Kompensation der Augenbewegung genutzt werden, wozu weitere Messwerte, wie die Vorderkammertiefe, Hornhautradius und Achslänge Verwendung finden.

Weiterhin lässt sich aus einem genügend breiten, 3-dimensionalen retinalen OCT-Scan der Fixationsfehler bestimmen. Auch hierzu werden weitere Messwerte, wie die Vorderkammertiefe, Hornhautradius und Achslänge genutzt.

Hierbei wird aus der lateralen Verschiebung der fovealen Grube und der Achslänge der Fixationsfehler zum Zeitpunkt dieser Datenaufnahme ermittelt. Damit lässt sich ebenso wie im vorherigen Absatz beschrieben der Fixationsfehler zu diesem Zeitpunkt kompensieren.

Mit der erfindungsgemäßen Lösung wird ein Verfahren zur Erfassung biometrischer Messdaten eines Patientenauges zur Verfügung gestellt, bei dem eine Kontrolle der Fixation während der gesamten biometrischen Messung möglich ist.

Dabei ist die Fixationskontrolle Teil der Messdatenaufnahme, um die Qualität der aufgenommenen Messdaten zu prüfen und idealerweise auch um Fixationsfehler zu kompensieren.

Die Fixationsprüfung und/oder Fixationskompensation erfolgt durchgehend und zwar ohne, dass dadurch die Messdatenaufnahme wesentlich verlängert wird.

Der entscheidende Vorteil der vorgeschlagenen Lösung liegt in der zeitlichen Übertragung der absoluten Fixationsinformation, durch eine zeitliche Verzahnung verschiedener Modalitäten der Datenaufnahme.

Dies erfolgt durch Ergänzung der absoluten Fixationsinformation aus einem zentralen retinalen OCT-Scan durch relative Fixationsinformationen aus anderen Modalitäten, die fortlaufend parallel zu oder zeitlich engmaschig abwechselnd mit anderen Datenaufnahmemodi durchgeführt werden.

Für die Art der Datenaufnahme mit relativer Fixationsinformation dürfte es mehrere Möglichkeiten geben. Entscheidend ist hierbei, dass Strukturen in verschiedenen Abständen vom Messgerät vorhanden sind, die sich bei Rotation des Auges im Kamerabild lateral relativ zueinander bewegen.

Insbesondere kann die ermittelte Rotation z. B. durch rigide Registrierung von Freiformflächen aus Topographiemessungen der Hornhautvorderseite verfeinert werden. Dies ist insbesondere bei Patienten mit hinreichend komplexer Hornhaut, was z. B. in Form von Astigmatismus häufig der Fall ist.

Mit dem vorgeschlagenen Verfahren wird die Fixation während der gesamten biometrischen Messung kontrolliert, was zur Folge hat, dass für das Patientenauge nur solche biometrischen Messdaten verwendet werden, die bei korrekter Fixation erfasst worden sind.

Bei Mehrfachmessungen können aus den vorliegenden biometrischen Messwerten noch Mittelwerte gebildet werden, wodurch sich die Qualität der aufgenommenen Messdaten zusätzlich verbessern lässt.

## Patentansprüche

1. Verfahren zur durchgehenden Kontrolle der Fixation eines Patientenauges während der Erfassung dessen biometrischer Messdaten, bei dem in Abhängigkeit der unterschiedlichen Aufnahmemodi aus bereits vorhandenen bzw. zusätzlich erfasster Aufnahmen und/oder Daten Informationen zur Fixation extrahiert werden, **gekennzeichnet durch** die Verfahrensschritte:
a) aufnehmen eines zentralen retinalen OCT-Scans mit absoluter Fixationsinformation,
b) aufnehmen eines Frontalbildes mit relativer Fixationsinformation gleichzeitig bzw. unmittelbar vor oder nach dem zentralen retinalen OCT-Scan, bei zumindest teilweiser, diffuser Beleuchtung,
c) zeitlich möglichst durchgehend aufnehmen weiterer Frontalbilder mit relativer Fixationsinformation,
d) prüfen des Fixationszustandes anhand des zentralen retinalen OCT-Scans,
e) bei Vorliegen einer korrekten Fixation wird diese auch für das im Verfahrensschritt b) aufgenommene Frontalbild angenommen,
f) berechnen eines, die Abweichungen zu dem im Verfahrensschritt b) aufgenommen Frontalbild beschreibenden, Differenzvektors für jedes im Verfahrensschritt c) aufgenommene Frontalbild,
g) vergleichen der berechneten Differenzvektoren der im Verfahrensschritt c) aufgenommenen Frontalbilder mit einem definierten Schwellwert, wobei
h) bei unterschrittenen Schwellwerten für die aufgenommenen Frontalbilder eine korrekte Fixation und bei überschrittenen keine korrekte Fixation angenommen wird und
i) nur die biometrischen Messdaten verwendet werden, die kurz vor, während oder kurz nach den Frontalbildern mit korrekter Fixation erfasst wurden.

2. Verfahren nach Anspruch 1, **dadurch gekennzeichnet, dass** für das im Verfahrensschritt b) aufzunehmende Frontalbild ein zusätzlicher Reflex auf der Kornea erzeugt wird.

3. Verfahren nach den Ansprüchen 1 und 2, **dadurch gekennzeichnet, dass** dieser zusätzlich erzeugte Reflex bevorzugt nahe am Vertex und besonders bevorzugt am Vertex erzeugt wird.

4. Verfahren nach den Ansprüchen 1 bis 3, **dadurch gekennzeichnet, dass** die Projektion des zusätzlichen Reflexes auf der Kornea mittels eines kollimierten Lichtstrahles entlang der optischen Achse des Gerätes erfolgt.

5. Verfahren nach Anspruch 1, **dadurch gekennzeichnet, dass** die Frontalbilder gemäß dem Verfahrensschritt c) in Abhängigkeit der Aufnahmemodi bei unterschiedlichen Beleuchtungssituationen aufgenommen werden.

6. Verfahren nach Anspruch 1, **dadurch gekennzeichnet, dass** im Ergebnis des Verfahrensschrittes d) eine korrekte Fixation vorliegt, wenn der zentrale retinale OCT-Scan die foveale Grube enthält.

7. Verfahren nach Anspruch 6, **dadurch gekennzeichnet, dass** der Bediener einen Hinweis erhält, wenn im Ergebnis des Verfahrensschrittes d) der zentrale retinale OCT-Scan die foveale Grube nicht enthält und keine korrekte Fixation vorliegt.

8. Verfahren nach Anspruch 7, **dadurch gekennzeichnet, dass** das Verfahren durch den Bediener oder automatisch erneut mit Verfahrensschritt a) beginnt.

9. Verfahren nach Anspruch 1, **dadurch gekennzeichnet, dass** für die Berechnung der Differenzvektoren nach Verfahrensschritt f) ein Kreisring an den Limbus oder die Pupille gefittet wird.

10. Verfahren nach Anspruch 9, **dadurch gekennzeichnet, dass** für die Berechnung der Differenzvektoren nach Verfahrensschritt f) der Abstand zwischen dem Mittelpunkt des Kreisringes und dem zusätzlich erzeugten Reflex genutzt wird.

11. Verfahren nach Anspruch 10, **dadurch gekennzeichnet, dass** die zu berechnenden Differenzvektoren von der Vorderkammertiefe des betreffenden Auges abhängig sind.

12. Verfahren nach Anspruch 1, **dadurch gekennzeichnet, dass** die berechneten Differenzvektoren zur Kompensation der Augenbewegung genutzt werden.

13. Verfahren nach Anspruch 12, **dadurch gekennzeichnet, dass** für die Kompensation der Augenbewegung weitere Messwerte, wie die Vorderkammertiefe, Hornhautradius und Achslänge genutzt werden.

14. Verfahren nach Ansprüchen 1 und 6, **dadurch gekennzeichnet, dass** aus einem genügend breiten, 3-dimensionalen retinalen OCT-Scan, der Fixationsfehler bestimmt wird.

15. Verfahren nach Anspruch 14, **dadurch gekennzeichnet, dass** basierend auf dem Fixationsfehler und weiteren Messwerten, wie die Vorderkammertiefe, Hornhautradius und Achslänge, die Augenbewegung kompensiert wird

## Claims

1. Method for monitoring the fixation of a patient's eye throughout the capture of its biometric measurement data, in which information relating to the fixation is extracted, depending on the different recording modes, from already available or additionally captured recordings and/or data, **characterized by** the method steps of:
a) recording a central retinal OCT scan with absolute fixation information,
b) recording a frontal image with relative fixation information at the same time as, or immediately before or after, the central retinal OCT scan with at least partly diffuse lighting,
c) recording further frontal images with relative fixation information as continuously as possible over time,
d) checking the fixation state on the basis of the central retinal OCT scan,
e) should a correct fixation be present, the latter is also assumed for the frontal image recorded in method step b),
f) calculating a difference vector for each frontal image recorded in method step c), said difference vector describing the deviations from the frontal image recorded in method step b),
g) comparing the calculated difference vectors of the frontal images recorded in method step c) with a defined threshold, wherein
h) a correct fixation for the recorded frontal images is assumed if the thresholds are undershot and no correct fixation is assumed if the thresholds are overshot and
i) use is only made of the biometric measurement data captured just before, at the same time as or just after the frontal images with the correct fixation.

2. Method according to Claim 1, **characterized in that** an additional reflection is generated on the cornea for the frontal image to be recorded in method step b).

3. Method according to Claims 1 and 2, **characterized in that** this additionally generated reflection is preferably generated near the vertex and particularly preferably generated at the vertex.

4. Method according to Claims 1 to 3, **characterized in that** the projection of the additional reflection on the cornea is implemented by means of a collimated light beam along the optical axis of the device.

5. Method according to Claim 1, **characterized in that** the frontal images are recorded as per method step c) depending on the recording modes at different lighting situations.

6. Method according to Claim 1, **characterized in that** a correct fixation is present in the result of method step d) if the central retinal OCT scan contains the foveal pit.

7. Method according to Claim 6, **characterized in that** the operator receives a notification if the central retinal OCT scan does not contain the foveal pit and there is no correct fixation in the result of method step d).

8. Method according to Claim 7, **characterized in that** the method is restarted with method step a), either by the operator or automatically.

9. Method according to Claim 1, **characterized in that** a circular ring is fitted to the limbus or the pupil for the calculation of the difference vectors as per method step f).

10. Method according to Claim 9, **characterized in that** the distance between the center of the circular ring and the additionally generated reflection is used for calculating the difference vectors as per method step f).

11. Method according to Claim 10, **characterized in that** the difference vectors to be calculated are dependent on the anterior chamber depth of the relevant eye.

12. Method according to Claim 1, **characterized in that** the calculated difference vectors are used to compensate the eye movement.

13. Method according to Claim 12, **characterized in that** further measurement values, such as the anterior chamber depth, corneal radius and axis length, are used for compensating the eye movement.

14. Method according to Claims 1 and 6, **characterized in that** the fixation error is determined from a sufficiently wide, 3-dimensional retinal OCT scan.

15. Method according to Claim 14, **characterized in that** the eye movement is compensated on the basis of the fixation error and further measurement values, such as the anterior chamber depth, corneal radius and axis length.

## Revendications

1. Procédé de contrôle continu de la fixation d'un œil de patient lors de l'acquisition de données de mesure biométriques de celui-ci, procédé dans lequel des informations sur la fixation sont extraites d'enregistrements et/ou de données déjà existants ou acquis en plus en fonction de différents modes d'acquisition, **caractérisé par** les étapes de procédé suivantes :
a) enregistrer un scan OCT rétinien central avec des informations de fixation absolues,
b) enregistrer une image frontale avec des informations de fixation relatives en même temps ou immédiatement avant ou après le scan OCT rétinien central, avec un éclairage diffus au moins partiel,
c) enregistrer d'autres images frontales avec des informations de fixation relatives autant que possible de manière temporellement continue,
d) vérifier l'état de la fixation à l'aide du scan OCT rétinien central,
e) en cas de fixation correcte, celle-ci étant également acceptée pour l'image frontale enregistrée à l'étape de procédé b),
f) calculer un vecteur différence, décrivant les écarts par rapport à l'image frontale enregistrée à l'étape de procédé b), pour chaque image frontale enregistrée à l'étape de procédé c),
g) comparer les vecteurs différences calculés des images frontales enregistrées à l'étape de procédé c) à une valeur seuil définie,
h) une fixation correcte étant acceptée si les images frontales enregistrées sont au-dessous des valeurs seuils et aucune fixation correcte n'étant acceptée en cas de dépassement, et
i) seules les données de mesure biométriques étant utilisées qui ont été enregistrées juste avant, pendant ou juste après les images frontales avec fixation correcte.

2. Procédé selon la revendication 1, **caractérisé en ce qu'**une réflexion supplémentaire est générée sur la cornée pour l'image frontale à enregistrer à l'étape de procédé b).

3. Procédé selon les revendications 1 et 2, **caractérisé en ce que** cette réflexion supplémentaire générée est de préférence générée à proximité du sommet et de manière particulièrement préférée au sommet.

4. Procédé selon les revendications 1 à 3, **caractérisé en ce que** la projection de la réflexion supplémentaire sur la cornée est effectuée au moyen d'un faisceau lumineux collimaté suivant l'axe optique de l'appareil.

5. Procédé selon la revendication 1, **caractérisé en ce que** les images frontales sont enregistrées pour différentes situations d'éclairage en fonction des modes d'enregistrement conformément à l'étape de procédé c).

6. Procédé selon l'une des revendications 1 à 6, **caractérisé en ce que** le résultat de l'étape de procédé d) est une fixation correcte si le scan OCT rétinien central contient la fosse fovéale.

7. Procédé selon la revendication 6, **caractérisé en ce que** l'opérateur reçoit une notification si, suite à l'étape de procédé d), le scan OCT rétinien central ne contient pas la fosse fovéale et s'il n'y a pas de fixation correcte.

8. Procédé selon la revendication 7, **caractérisé en ce que** le procédé recommence à l'étape de procédé a) automatiquement ou par le biais de l'opérateur.

9. Procédé selon l'une des revendications précédentes, **caractérisé en ce que** pour le calcul des vecteurs différences selon l'étape de procédé f), un anneau circulaire est monté sur le limbe ou la pupille.

10. Procédé selon la revendication 9, **caractérisé en ce que** la distance entre le point central de l'anneau et la réflexion supplémentaire générée est utilisée pour le calcul des vecteurs différences après l'étape de procédé f).

11. Procédé selon la revendication 10, **caractérisé en ce que** les vecteurs différences à calculer sont dépendants de la profondeur de la chambre antérieure de l'œil considéré.

12. Procédé selon la revendication 1, **caractérisé en ce que** les vecteurs différences calculés sont utilisés pour compenser le mouvement des yeux.

13. Procédé selon la revendication 12, **caractérisé en ce que** d'autres valeurs de mesure, telles que la profondeur de la chambre antérieure, le rayon cornéen et la longueur axiale, sont utilisées pour compenser le mouvement des yeux.

14. Procédé selon les revendications 1 et 6, **caractérisé en ce que** l'erreur de fixation est déterminée à partir d'un scan OCT rétinien tridimensionnel suffisamment large.

15. Procédé selon la revendication 14, **caractérisé en ce que** le mouvement des yeux est compensé sur la base de l'erreur de fixation et d'autres valeurs de mesure, telles que la profondeur de la chambre antérieure, le rayon cornéen et la longueur axiale.
